(19) Europäisches Patentamt
European Patent Office
Office européen des brevets

(11) Publication number: **0 194 710 B1**

(12) # EUROPEAN PATENT SPECIFICATION

(45) Date of publication of patent specification: **02.11.89**

(51) Int. Cl.$^4$: **C 07 H 11/04,** A 61 K 31/70

(21) Application number: **86200252.4**

(22) Date of filing: **19.02.86**

(54) **New derivative of fructose, its preparation and its uses.**

(30) Priority: **15.03.85 ES 541277**

(43) Date of publication of application:
**17.09.86 Bulletin 86/38**

(45) Publication of the grant of the patent:
**02.11.89 Bulletin 89/44**

(84) Designated Contracting States:
**DE FR GB IT**

(56) References cited:

**CHEMICAL ABSTRACTS, vol. 98, no. 9, 28th Februar 1983, abstract no. 70693a, Columbus, Ohio, US; S.A. PRASAD: "Experimental zinc deficiency in humans. An overview of original studies" & ACS SYMP. SER. 1983, 210 (NUTR. BIOAVAILABILITY ZINC), 1-14**

(73) Proprietor: **LABORATORIOS VINAS S.A.**
**Torrente Vidalet Street 29**
**Barcelona (ES)**

(72) Inventor: **Vinas, Antonio Buxadé**
**Provenza Street 278**
**Barcelona (ES)**

(74) Representative: **Schmitz, Yvon et al**
**Bureau Gevers S.A. Rue de Livourne 7, B.1**
**B-1050 Bruxelles (BE)**

(56) References cited:
**CHEMICAL ABSTRACTS, vol. 98, no. 3, 17th January 1983, page 253, abstract no. 13598z, Columbus, Ohio, US; G.B. VAN DEN BERG et al.: "Zinc, magnesium and hydrogen ion binding to D-fructose 1,6-bisphosphate studied by phosphorus-31 and proton NMR" & ARCH. BIOCHEM. BIOPHYS. 1982, 219(2), 268-76**

**CHEMICAL ABSTRACTS, vol. 106, no. 1, 5th January 1987, page 5364, abstract no. 5369u, Columbus, Ohio, US; & ES-A-541 277 (LABORATORIOS VINAS S A) 16-12-1985**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 194 710 B1

(56) References cited:

CHEMICAL ABSTRACTS, vol. 98, no. 9, 28th Februar 1983, abstract no. 70693a, Columbus, Ohio, US; S.A. PRASAD: "Experimental zinc deficiency in humans. An overview of original studies" & ACS SYMP. SER. 1983, 210 (NUTR. BIOAVAILABILITY ZINC), 1-14

CHEMICAL ABSTRACTS, vol. 98, no. 3, 17th January 1983, page 253, abstract no. 13598z, Columbus, Ohio, US; G.B. VAN DEN BERG et al.: "Zinc, magnesium and hydrogen ion binding to D-fructose 1,6-bisphosphate studied by phosphorus-31 and proton NMR" & ARCH. BIOCHEM. BIOPHYS. 1982, 219(2), 268-76

## Description

This invention relates to a new derivative of fructose, more precisely to the zinc salt of fructose-1,6-diphosphate of the formula:

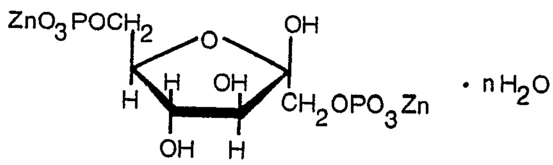

wherein n = 0 to 4.

This invention has a lot of advantages for applications which can be deduced from the formula, on a therapeutical point of view.

As a matter of fact, the structure such as obtained comprises in its formula the divalent cation *zinc* ($Zn^{+2}$) and the *fructose-1,6-diphosphate,* whereby administration of the product — zinc fructose-1,6-diphosphate — in dosages which may vary in accordance with a galenic formulation suitable to the selected administration route (oral, parenteral, rectal and the like administration) allows the animal system (experimental pharmacology) or humans to dispose of both elements with their resulting advantages.

*Zinc* exerts therapeutical effects in gonadal dysfunction. The plasma concentration of zinc is an important factor in the fertility regulation; decrease of potential fertility of the man and secretory male sterility when Zn levels in seminal plasma are low have been described. Addition of zinc normalizes serum testosterone and concentration of spermatozoa in ejaculations and motility of the same, which is considered as being necessary as a favourable medium for motility and activity of the spermatozoa. Zinc bioavailability influences the activity of some testicular enzymes.

Medical publications report cases of idiopathic male infertility, of involution of testicular function with age successfully treated with Zn. There were zome cases of women who were pregnant after therapeutic treatment of the affected man.

In many mammals, including humans, seminal plasma contains *fructose* as main glucose. In men, seminal fructose mainly derives from seminal vesicles.

Fructose concentration in human ejaculation is of about 100—500 mg/100 cc. Fructose supplies necessary energy to spermatozoa for their motility by anaerobic glycolysis. It is the substrate for spermatozoa metabolism. The number of mg of fructose used by spermatozoa after one hour of incubation at 37°C can be measured and the basic function of this glucose proves correct. Mature human spermatozoa fail to be functional if they cannot use fructose as main source of energy for production of ATP.

It has been proved that products causing male infertility, for example, chlorohydrins, inhibit oxidizing metabolism of fructose.

There are deficits of the sperm in fructose, which are associated with some forms of hypogonadism. Ejaculations in which the seminal plasma has not been sufficiently renewed (24 hours) only contain a small amount of fructose. The determination of fructose in ejaculation not only provides a quantitative measure of the vesicular contribution to the ejaculation constitution but can also be used as a useful diagnosis means for detecting an occlusion of ejaculation ducts.

The fructose derivative such as obtained has as an advantage in comparison with other zinc salts, on the toxicological point of view, its low acute toxicity orally, a dosage of 4000 mg/kg causing no mortality in a group of 10 rats as it has been verified. Intraperitoneally, the $LD_{50}$ value has been rated between 125—150 mg/kg (of the same order as for other zinc salts). This low oral toxicity is in relation with the slow absorption that this molecule presents and is particularly interesting for its administration through this route. It has been verified that the zinc values in plasma start to increase two hours after administration of the product. Five hours after administration, increases of the order of 40% of the $Zn^{2+}$ content in this plasma have been rated.

Depending on the pathology to which the product could be applied, levels of $Zn^{2+}$ in seminal vesicle and testicles were determined in treated rats in comparison with controls.

In the enclosed table, levels of $Zn^{2+}$ in ppm referred to the organ weight are indicated. One can appreciate an increase of $Zn^{2+}$ levels as well in testicles as in siminal vesicle. They represent average values (with mean errors).

The process of the present invention is characterised by reaction of fructose-1,6-diphosphate or of its ammonium salt or of an alkaline metal salt thereof with an oxide, hydroxide or salt of zinc in a polar solvent or solvent mixture, preferably water or methanol. The reaction temperature may vary between 0°C and the boiling point of the solvent. By cold cristallization or by elimination of the solvent, zinc fructose-1,6-diphosphate is obtained.

In order to facilitate the explanation, some examples of carrying out the process of the invention are described hereinafter, these examples being of an illustrative but non limitative character.

Example 1

In a 250 ml flask, 8.98 g of disodium fructose-1,6-diphosphate are dissolved in 40 ml of water, a previously prepared solution of 8.78 g of monohydrate zinc acetate in 100 ml of water is added with stirring, the solution is brought to a temperature of 60°C and maintained under a good stirring for 1 hour, then allowed to cool and maintained at 5°C overnight, a crystalline precipitate is obtained and filtered, then washed with water and dried in a desiccator, obtaining 7.15 g of product. The combined mother liquids are treated with acetone, producing a new precipitate which is treated as previously, 2.04 g of additional product being obtained. M.P. >300°C.

Elementary analysis

Calculated for $C_6H_{14}O_{14}P_2Zn_2$: C: 14.33%; H: 2.81%; Zn: 26.00%.

Found: C: 14.03%; H: 2.67%; Zn: 26.0%.

The IR spectrum is analogous to that of disodium fructose-1,6-diphosphate.

Example 2

In a 250 ml flask, 8.98 g of disodium frustose-1,6-diphosphate are dissolved with 140 ml of water, 4.39 g of basic zinc carbonate are added with stirring, the solution is brought to 60°C and maintained under stirring for 1 hour, obtaining a solution which is treated as in the preceding Example. 9.30 g of a product of characteristics analogous to those of Example 1 are obtained.

| Table | Control | Treated |
|---|---|---|
| Seminal vesicle | 500.6 ± 51.2 | 651.2 ± 34.3* |
| Testicle | 680.8 ± 43.6 | 726 ± 24.4 |

*p<0.05.

**Claims**

1. Zinc fructose-1,6-diphosphate of the formula:

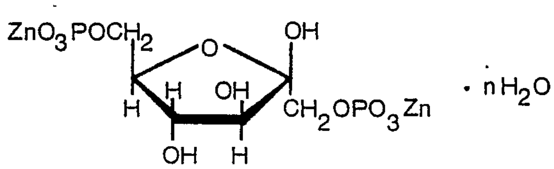

wherein n = 0 to 4.

2. Process for preparing zinc fructose, 1,6-diphosphate of the formula given in claim 1, characterized by reaction of a zinc oxide, hydroxide or salt with fructose-1,6-diphosphate or an ammonium salt or alkaline metal salt thereof in a polar solvent or mixture of solvents at a temperature comprised between 0°C and the boiling point of the solvent.

3. Zinc fructose-1,6-diphosphate of the formula given in claim 1, for use as a therapeutic agent, in particular in male infertility and in involution of testicular function with age.

**Patentansprüche**

1. Zinkfruktose 1,6-Diphosphat mit der Strukturformel:

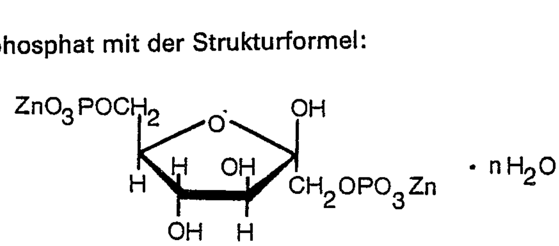

wobei n = 0 bis 4 ist.

2. Verfahren zum Herstellen von Zinkfruktose 1,6-Diphosphat mit der Formel, wie sie in Anspruch 1 angegeben ist, dadurch gekennzeichnet, daß Zinkoxid, Hydroxid oder Salz mit Fruktose 1,6-Diphosphat oder einem Ammoniumsalz oder Alkalimetallsalz davon in einem polaren Lösungsmittel oder einer Mischung von Lösungsmitteln bei Temperaturen von zwischen 0°C und dem Siedepunkt des Lösungsmittels reagiert.

3. Zinkfruktose 1,6-Diphosphat nach Anspruch 1, zur Verwendung als therapeutischer Wirkstoff, insbesondere bei männlicher Unfruchtbarkeit und bei Rückbildung der Hodenfunktion mit dem Alter.

**Revendications**

1. Fructose-1,6-diphosphate de zinc de la formule:

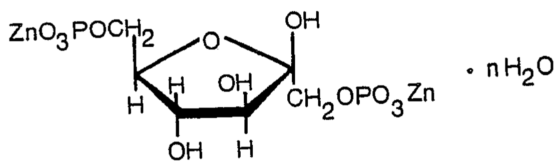

dans laquelle n = 0 à 4.

2. Procédé de préparation de fructose-1,6-diphosphate de zinc de la formule donnée dans la revendication 1, caractérisé par la réaction d'un oxyde, hydroxyde ou sel de zinc avec du fructose-1,6-diphosphate ou un sel d'ammonium ou sel de métal alcalin de celui-ci dans un solvant ou mélange de solvants polaires à une température comprise entre 0°C et le point d'ébullition du solvant.

3. Fructose-1,6-diphosphate de zinc de la formule donnée dans la revendication 1, utilisable comme agent thérapeutique, en particulier dans l'infertilité mâle et dans l'involution de la fonction testiculaire en fonction de l'âge.